# EUROPÄISCHE PATENTSCHRIFT

(11) **EP 1 419 167 B1**
(45) Veröffentlichungstag und Bekanntmachung des Hinweises auf die Patenterteilung: **16.02.2005**
(21) Anmeldenummer: 02796259.6
(22) Anmeldetag: 21.08.2002
(51) Int. Cl.: C07H 19/06, C07D 498/18

(54) **1-(2,5'-ANHYDRO-LYXOFURANOSYL) THYMINE ZUR SYNTHESE VON RADIOMARKIERTEN FLUOR-3' DEOXYNUCLEOTIDEN**
1-(2,5'-ANHYDRO-LYXOFURANOSYL) THYMINES FOR THE SYNTHESIS OF RADIOMARKED FLUORO-3' DEOXYNUCLEOTIDES
1-(2,5'-ANHYDRO-LYXOFURANOSYL) THYMINES UTILISEES POUR LA SYNTHESE DE FLUOR-3' DESOXYNUCLEOTIDES RADIO-MARQUES

(30) Priorität: 23.08.2001 DE 10141300
(43) Veröffentlichungstag der Anmeldung: 19.05.2004
(73) Patentinhaber: Deutsches Krebsforschungszentrum Stiftung des öffentlichen Rechts, 69120 Heidelberg (DE)
(72) Erfinder: EISENBARTH, Josef, Antonius, Maria, 68775 Ketsch (DE); MARTIN, Stefan, Johannes, 69221 Dossenheim (DE); WAGNER-UTERMANN, Ulrike, 64646 Heppenheim (DE); EISENHUT, Michael, 69118 Heidelberg (DE)
(74) Vertreter: Störle, Christian, Dr.
(86) Internationale Anmeldenummer: PCT/EP2002/009361
(87) Internationale Veröffentlichungsnummer: WO 2003/018599

(56) Entgegenhaltungen:
- MINAMOTO, KATSUMARO ET AL: "Synthesis and alkali-hydrolysis reactions of some 2,3'-(substituted imino)pyrimidine nucleosides lacking a 2'-hydroxyl group" JOURNAL OF ORGANIC CHEMISTRY (1989), 54(19), 4543-9 , XP001119673
- GRIERSON J R ET AL: "Radiosynthesis of 3'-deoxy-3'-[F]fluorothymidine: [F]FLT for imaging of cellular proliferation in vivo" NUCLEAR MEDICINE AND BIOLOGY, ELSEVIER SCIENCE PUBLISHERS, NEW YORK, NY, US, Bd. 27, Nr. 2, Februar 2000 (2000-02), Seiten 143-156, XP004196582 ISSN: 0969-8051 in der Anmeldung erwähnt
- MACHULLA H J ET AL: "SIMPLIFIEDLABELING APPROACH FOR SYNTHESIZING 3'-DEOXY-3'-15FFLUOROTHYMIDINE(18FFLT)" JOURNAL OF RADIOANALYTICAL AND NUCLEAR CHEMISTRY, ARTICLES, ELSEVIER SEQUOIA S.A., LAUSANNE, CH, Bd. 243, Nr. 3, 2000, Seiten 843-846, XP008003386 in der Anmeldung erwähnt
- WODARSKI C ET AL: "SYNTHESIS OF 3'-DEOXY-3'-18FFLUORO-THYMIDINE WITH 2,3'-ANHYDRO-5'-O-(4,4'-DIMETHOXYTRITYL)-T HYMIDINE" JOURNAL OF LABELLED COMPOUNDS AND RADIOPHARMACEUTICALS, SUSSEX, GB, Bd. 43, Nr. 12, 30. Oktober 2000 (2000-10-30), Seiten 1211-1218, XP008003378 ISSN: 0362-4803 in der Anmeldung erwähnt

## Beschreibung

Die Erfindung bezieht sich auf Verbindungen, die als markierungsfähige Vorläufersubstanzen zur Herstellung von insbesondere 3'-[¹⁸F]Fluor-3'-deoxynucleotiden geeignet sind, Verfahren zu deren Herstellung und deren Verwendung zur Synthese von insbesondere 3'-[¹⁸F]Fluor-3'-deoxynucleotiden.

In der Nuklearmedizin können mit Hilfe der sogenannten Positronen-Emissions-Tomographie (PET) Wirkungsweisen von körpereigenen oder -fremden Stoffen, z. B. Pharmaka, Stoffwechselvorgänge im Gehim und anderen Organen, insbesondere in Tumoren, untersucht werden. Gerade in der Tumordiagnostik wird PET häufig eingesetzt. Dazu werden biologisch relevante, mit positronenemittierenden Radionukliden markierte Verbindungen injiziert und die emittierte γ-Strahlung in Tomogrammen erfaßt.

Als markierte Verbindungen können dabei 3'-Deoxynucleotide eingesetzt werden, die an 3'-Position ein positronenemittierendes Radionukild, z. B. ¹⁸F, aufweist.

Beispiel einer solchen markierten Verbindung ist 3'-[¹⁸F]Fluor-3'-deoxythymidin([¹⁸F]FLT), 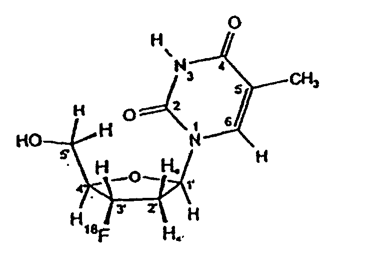 das sich in schnell teilenden Zellen, wie sie bei Tumoren vorliegen, anreichert Auf diese Weise lassen sich zum Beispiel Tumore, besonders im Hirn, aber auch im Körperstamm, lokalisieren oder therapiebegleitend das Ansprechen auf eine Behandlung beurteilen oder deren Optimierung veranlassen.

Die Herstellung von [¹⁸F]FLT verläuft bisher über mehrere Zwischenstufen, bei denen hochtoxische Chemikalien eingesetzt werden oder entstehen, die dann vor der Verabreichung des Proliferationsmarkers [¹⁸F]FLT vollständig abgetrennt werden müssen, damit sie den zu untersuchenden Probanden nicht schädigen.

So beschreiben Grierson J. R. und Shields A. F. in *Nucl*. *Med*. *Biol.,* 2000, *27*, 143 - 156 eine aufwendige Synthese von Markierungsvorläufem für die Herstellung von [¹⁸F]FLT, wobei zum Teil hochtoxische Verbindungen, wie Phosgen, verwendet werden. Dabei wird mit Dimethoxybenzyl-*N*-Schutzgruppen gearbeitet, die oxidativ mit Cer(IV)-Ammoniumnitrat abgespaltet werden müssen. Cer-Verbindungen sind aber toxisch, weshalb sie in einer reproduzierbaren Fällungsreaktion quantitativ abgetrennt werden müssen. Diese Arbeitsabläufe werden in der Regel manuell durchgeführt, was aber aus Strahlenschutzgründen wiederum vermieden werden soll, weil die Strahlenbelastung und die Gefahr der Kontamination von Menschen so niedrig wie möglich gehalten werden soll.

Machulla et al. in *J*. *Radioanal*. *Nucl*. *Chem.,* 2000, *243*, 843 - 846 und Wodarski et al. in *J*. *Labelled Cpd Radiopharm,* 2000, *43*, 1211 - 1218 beschreiben die Synthese von Markierungsvortäufern für die Herstellung von [¹⁸F]FLT, wobei aber extreme Reaktionsbedingungen angewandt werden, beispielsweise Reaktionstemperaturen von etwa 160°C, und DMSO als Lösungsmittel eingesetzt wird. Dies läßt eine Umsetzung der Synthese auf kommerziell erhältliche Synthesemodule wenn überhaupt nur eingeschränkt zu. Des weiteren ist DMSO als hochsiedendes Lösungsmittel nur sehr schwer entfembar.

Der vorliegenden Erfindung liegt somit die Aufgabe zugrunde, eine markierungsfähige Verbindung für die Synthese von 3'-Deoxynucleotiden, die an 3'-Position ein positronenemittierendes Radionucleotid, wie ¹⁸F, aufweisen bereitzustellen, die nicht die aus dem Stand der Technik bekannten Nachteile aufweisen.

Erfindungsgemäß wird dies durch eine Verbindung erreicht, die sich dadurch auszeichnet, daß sie die Struktur der Formel (1) aufweist 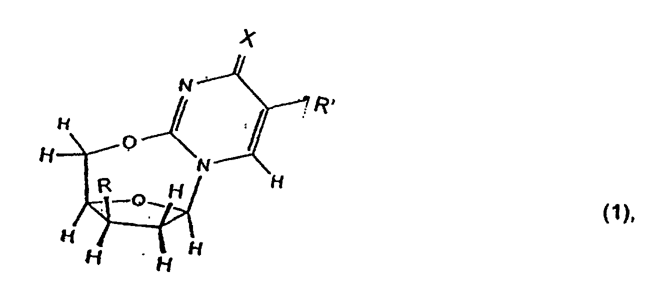 in der
R für Br, J oder R¹-SO₃, wobei R¹ ein unsubstituiertes oder substituiertes C₁-C₅-Alkyl oder ein unsubstituiertes oder substituiertes Phenyl bedeutet;
X für O oder NR", wobei R" eine übliche Schutzgruppe für N bedeutet, und
R' für Wasserstoff, Halogen, wie F, CI oder Br, jeweils substituiertes oder unsubstituiertes C₁-C₇-Alkyl, wie Methyl oder Ethyl, C₂-C₇-Alkenyl oder C₂-C₇-Alkinyl steht.

Bei "üblichen Schutzgruppen" im Sinne der vorliegenden Erfindung handelt es sich um solche organische Reste, mit denen Amino-Funktionen vorübergehend gegen den Angriff von Reagenzien geschützt werden können. Dem Fachmann sind solche üblichen Schutzgruppen für Amino-Funktionen bekannt. Es handelt sich beispielsweise um die Benzyloxycarbonyl-, die tert.Butoxycarbonyl-, die 9-Fluorenylmethoxycarbonyl-, die Triphenylmethyl- oder die Nitrobenzolsulfenyl-Gnuppe.

Bei den am C₁-C₇-Alkyl, C₂-C₇-Alkenyl oder C₂-C₇-Alkinyl vorliegenden Substituenten kann es sich um Halogene, wie F, Cl, Br, NO₂, Ph₃Sn, Bu₃Sn, Me₃Sn, Ph₃Si, Bu₃Si und/oder Me₃Si handeln.

Gemäß der vorstehenden Formel (1) steht R für R¹-SO₃, wobei R¹ ein unsubstituiertes oder substituiertes C₁-C₅-Alkyl sein kann. Beispiel eines unsubstituierten C₁-C₅-Alkyl ist Methyl, d. h. R¹-SO₃ umfaßt Methansulfonyl. Diese sind günstig, da die Reaktion, mit der sie in die erfindungsgemäße Verbindung eingeführt werden, z.B. die Mesylierung, schnell abläuft und die erfindungsgemäße Verbindung in hohen Ausbeuten erhalten wird.

Der Substituent am C₁-C₅-Alkyl ist vorzugsweise eine elektronenziehende Gruppe, damit der Rest R eine gute Abgangsgruppe darstellt. Beispiele von elektronenziehenden Gruppen sind Halogene, wie F, Cl, Br und I, sowie NO₂, wobei wegen der günstigen elektronenziehenden Eigenschaft Fluor besonders geeignet ist Beim substituierten C₁-C₅-Alkyl kann 1 H-Atom bis alle der H-Atome des Alkyl-Restes durch einen elektronenziehenden Substituenten ersetzt sein, die unabhängig voneinander gewählt werden können. Ein besonders bevorzugter Vertreter des substituierten C₁-C₅-Alkyl-Rests ist CF₃, d. h. R¹-SO₃ umfaßt Trifluormethansulfonyl, das eine besonders gute Abgangsgruppe darstellt.

Wie bereits vorstehend ausgeführt wurde, kann der Rest R¹ ein unsubstiuiertes oder ein substituiertes Phenyl sein. Dabei kann das Phenyl 1 oder mehrere Substituenten aufweisen, die gleich oder voneinander verschieden sein können. Die Substituenten können sich in o-, m- und/oder p-Position zum SO₃-Rest befinden. Beispiele geeigneter Substituenten sind C₁-C₅-Alkyl-Reste, wie Methyl, oder elektronenziehende Gruppen, insbesondere Halogene, wie F, Cl, Br und I, sowie die NO₂-Gruppe, wodurch der Rest R eine gute Abgangsgruppe darstellt. Bevorzugte Reste R¹-SO₃ mit substituiertem Phenyl umfassen 4-Nitrophenytsulfonyl oder p-Toluensulfonyl, die besonders gute Abgangsgruppen sind.

Besonders bevorzugte erfindungsgemäße Verbindung sind 1-(3'-O-Mesyl-2,5'-anhydro-β-Dlyxofuranosyl)thymin, 1-(3'-*O*-mesyl-2,5'-anhydro-β-D-lyxofuonosyl)uridin und 1-(3'-*O*-mesyl-2,5'anhydro-β-D-lyxofuranosyl)cytidin.

Überraschenderweise wurde nun festgestellt, daß ausgehend von den erfindungsgemäßen Verbindungen in einfacher und schneller Weise die in der Positronen-Emissions-Tomographie verwendete Verbindung [¹⁸F]FLT oder andere an 3'-Position mit einem positronenemittierenden Radionukleid markiert 3'-Deoxynukleotid hergestellt werden können. Mit den erfindungsgemäßen Verbindungen stehen Vorläufersubstanzen zur Synthese von Proliferationsmarkers, wie [¹⁸F]FLT, zur Verfügung, die aufgrund der intramolekularen Schützung nicht zu derivatisierender funktioneller Gruppen (5'-*O* und 3-*N*-Position) bei der Hydrolyse zum Zielmolekül z. B. ([¹⁸F]FLT) keine toxischen Produkte aus der Abspaltung der Schutzgruppen freisetzen. Dies vereinfacht die Aufarbeitung der Reaktionslösung und die Validierung des Syntheseprozesses zu einem GMP/GLP-gerechten, applizierbaren Radiopharmakon. Die Radiomarkierung z. B. zu [¹⁸F]FLT verläuft einfach, schnell, in guten Ausbeuten, hoher Reinheit und unter milden Bedingungen.

Die bei den erfindungsgemäßen Verbindungen eingeführte Anhydrostruktur ersetzt die ansonsten notwendige Verwendung von Schutzgruppen in den Positionen N³ und 5'. Die intramolekulare Schützung derjenigen funktionellen Gruppen, die nicht derivatisiert werden sollen, ist in dieser Zusammenstellung bisher nicht beschrieben. Dieser intramolekulare Schutz verhindert vollständig die Bildung abgespaltener Schutzgruppenprodukte bei der basischen Hydrolyse der Anhydrostruktur, die bei den bisherigen Verfahren während der Hydrolyse der Vorläuferverbindung in äquimolarer Soffmenge entstehen.

Gegenstand der vorliegenden Erfindung ist ferner ein Verfahren zur Herstellung der erfindungsgemäßen Verbindung der Formel (1), wobei in eine Verbindung der Formel (2) 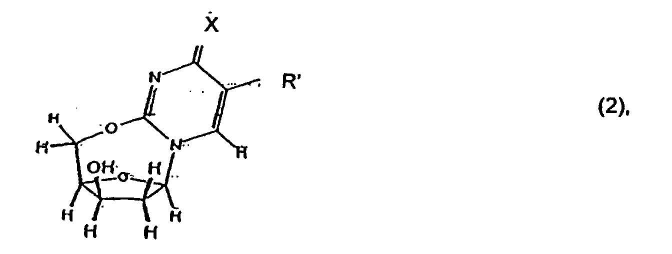 in der X und R' wie vorstehend definiert sind, der Rest R eingeführt wird, wobei R die vorstehenden Bedeutungen hat.

Das Einführen von R kann, abhängig von der chemischen Natur von R, in an sich bekannter Weise erfolgen. Solche Verfahren sind dem Fachmann bekannt; er kennt hierzu notwendige Reaktionsbedingungen, Chemikalien und Geräte.

Beispielsweise kann R¹-SO₂ in die erfindungsgemäße *Verbindung* eingebracht werden, indem die Verbindung (2) mit R¹-SO₂Hal, wobei R¹ wie vorstehend definiert ist und Hal für ein Halogen, insbesondere Chlor, steht, umgesetzt wird. Beispiele für R¹-SO₂Hal sind Methansulfonylchlorid, 4-Nitrophenylsulfonytchlocid, p-Toluensulfonylchlorid und Trifluormethansulfonsäurechlorid. Diese Umsetzung kann in günstiger Weise bei moderater Temperatur durchgeführt werden. Als Lösungsmittel kann trockenes Pyridin oder ein trockenes Gemisch von Triethylamin/Dichlormethan eingesetzt werden. Die Verbindung der Formel (2) kann in dem Lösungsmittel vorgelegt und dazu die Verbindung der Formel R¹-SO₂Hal zugegeben werden.

Die Isolierung und Reinigung der erfindungsgemäßen Verbindung kann in üblicher Weise erfolgen. Dazu kann beispielsweise das Lösungsmittel unter vermindertem Druck entfernt und das Rohprodukt chromatographisch, beispielsweise an Kieselgel, gereinigt werden.

Die Verbindung der Formel (2) kann ausgehend von Thymidin, Uridin oder Cytidin durch Ausbildung der intramolekularen Etherbildung, Einführung der Schutzgruppe R und Konfigurationsumkehr hergestellt werden. Diese Reaktionen sind dem Fachmann bekannt, d. h. er kennt hierzu notwendige Reaktionsbedingungen, Chemikalien und Geräte.

Ein Syntheseweg für die erfindungsgemäße Verbindung, ausgehend von Thymidin, ist zusammenfassend nachfolgend dargestellt, wobei das erfindungsgemäße Verfahren den letzten Schritt darstellt Dieses Verfahren kann auch analog für die Herstellung der erfindungsgemäßen Verbindung ausgehend von Uridin oder Cytidin angewandt werden.

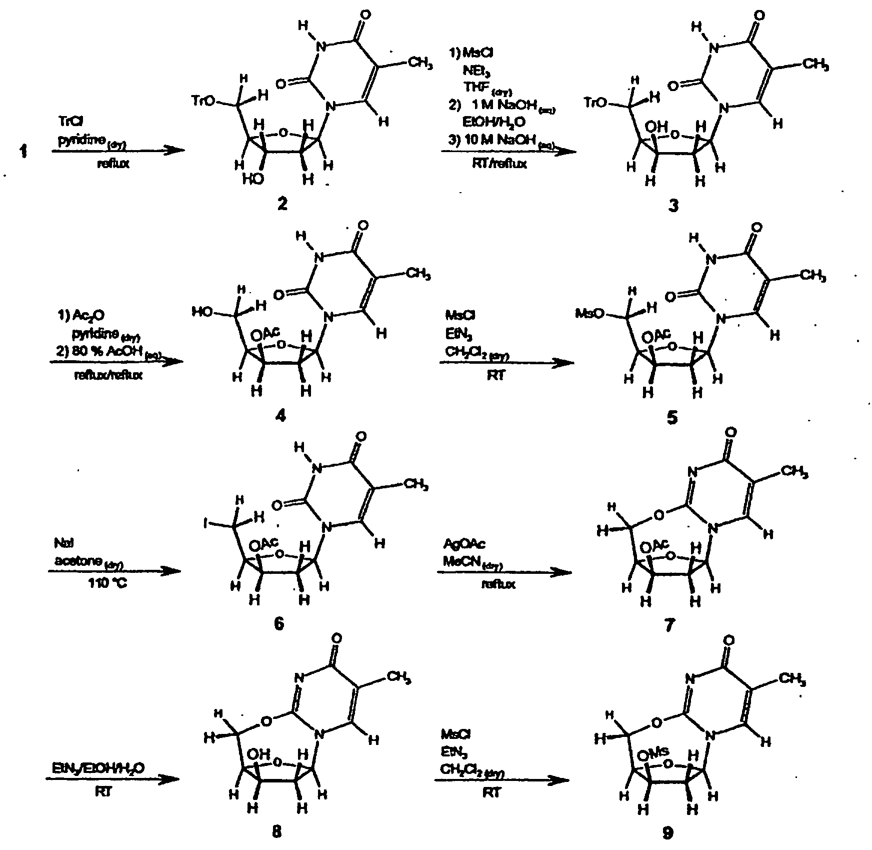

Dabei besitzt der Rest R die vorstehenden Bedeutungen. In diesem Reaktionsschema sind günstige Reaktionsbedingungen der einzelnen Schritte mit üblichen Abkürzungen wiedergegeben. Mit 1 wird die Ausgangsverbindung Thymidin bezeichnet. Der Syntheseweg von 1 zur erfindungsgemäßen Verbindung umfaßt acht Stufen. Hiervon sind die Einführung der Trityl- und der Acetylschutzgruppe und die Konfigurationsumkehr literaturbekannt.

Stehen kommerziell erhältliche Produkte zur Verfügung, die in vorstehendem Syntheseschema als Zwischenstufen aufgeführt sind, kann die Synthese der erfindungsgemäßen Verbindung natürlich ausgehend von den kommerziellen Verbindungen beginnen.

Das erfindungsgemäße Verfahren weist eine Reihe von Vorteilen auf. So werden als Edukte bzw. Lösungsmittel handelsübliche Reagenzien verwendet, die schnell und preisgünstig zur Verfügung stehen. Des weiteren werden allgemein übliche Laborpraktiken eingesetzt, wie Umsetzung bei Raumtemperatur, Durchführung der Reaktionen mit üblichen Rührem, Verwendung von Inertgas und chromatographische Verfahren zur Isolierung und Reinigung von Produkten. Die Reaktionstemperatur beim erfindungsgemäßen Verfahren ist niedrig, so daß keine aufwendigen Vorrichtungen zum Erhitzen erforderlich sind. Die beim erfindungsgemäßen Verfahren eingesetzten Lösungsmittel sind im allgemeinen niedrig siedend und können somit leicht abgetrennt werden. Des weiteren können im erfindungsgemäßen Verfahren Lösungsmittel eingesetzt werden, wie Acetonitril, die im Vergleich zu DMSO die Trennung mittels HPLC weniger stören. Das erfindungsgemäße Verfahren ist automatisierbar, so daß es aus strahlenschutztechnischen Gründen günstig ist, da die Strahlenbelastung und die Kontamination von Menschen so gering wie möglich gehalten wird. Ausgehend von den mit dem erfindungsgemäßen Verfahren hergestellten erfindungsgemäßen Verbindungen können in einfacher und schneller Weise in der Positronen-Emissions-Tomographie verwendete mit einem positronenemittierenden Radionukleid markierte Verbindungen, z. B. [¹⁸F]FLT hergestellt werden, die in hoher Ausbeute und in hoher Reinheit erhältlich sind. Trotz des vordergründig aufwendig erscheinenden Syntheseweges sind die einzelnen Stufen schnell und in guten Ausbeuten zu realisieren. Mit dem erfindungsgemäßen Verfahren ist eine gegenüber anderen Verfahren konkurrenzfähige Synthese in bezug auf die Ausbeute und Reinheit von z. B. [¹⁸F]FLT gegeben und ist überlegen im Hinblick auf die Bildung toxischer Hydrolyseprodukte.

Gegenstand der vorliegenden Erfindung ist ferner die Verwendung der erfindungsgemäßen Verbindung zur Einfügung eines Nucleophils an 3'-Position Bei Nucleophilen handelt es sich um "kemsuchende" Teilchen, wie Anionen, Carbanionen oder Lewis-Basen, die eine elektrophile Verbindung angreifen. Diese Nucleophile können positronenemittierende Radionuklide sein. Beispiel eines Nucleophils ist ¹⁸F. Nachfolgend wird zur Veranschaulichung dieser Einfügung die Herstellung von [¹⁸F]FLT beschrieben. Der Fachmann ist aufgrund dieser Informationen in der Lage, auch andere Nucleophile in die erfindungsgemäße Verbindung einzufügen.

Bei der Herstellung von [¹⁸F]FLT wird in einer erfindungsgemäßen Verbindung der Rest R durch ¹⁸F substituiert, es erfolgt dabei eine Konfigurationsumkehr, und anschließend wird die Anhydrostruktur hydrolysiert. Da in der erfindungsgemäßen Verbindungen aufgrund der intramolekularen Cyclisierung keine Schutzgruppen vorliegen, ist eine einfache basische Hydrolyse der markierten Zwischenverbindung in homogener Lösung zum gewünschten Produkt [¹⁸F]FLT möglich, ohne daß toxische Schutzgruppenhydrolysate abgespalten werden. Die Hydrolyse kann sauer oder basisch erfolgen. Hierbei kommt beispielsweise Natronlauge oder Salzsäure zum Einsatz, die nach der Neutralisation das untoxische Natriumchlorid (Kochsalz) bildet, wobei dieses durch die nachfolgende Reinigung, z.B. mittels HPLC, abgetrennt werden kann.

Während der Markierungsreaktion kann die erfindungsgemäße Verbindung mit [¹⁸F]Fluorid in einem organischen Lösungsmittel, insbesondere Acetonitril, in Gegenwart einer Base, wie Kaliumcarbonat, und eines makrocyclischen Neutral-Liganden, wie Kryptofix® 222 der Firma Merck, zu dem durch die Anhydrostruktur geschützten [¹⁸F]FLT umgesetzt werden, die hydrolysiert wird. Das Rohprodukt [¹⁸F]FLT kann chromatographisch gereinigt werden, z. B. mit einer Aufreinigungs-Kartusche und/oder mittels HPLC (z. B. Laufmittel: H₂O:Ethanol = 92,5:7,5, isokratisch; Säule: Phenomenex LUNA 5 µ 250 x 4,6 mm).

Mit der erfindungsgemäßen Verwendung ist es in überraschend einfacher und schneller Weise unter milden Bedingungen in guten Ausbeuten und in hoher Reinheit möglich, Nucleophile einzufügen, insbesondere Proliferationsmarker herzustellen. Dieses Herstellungsverfahren ist automatisierbar, d. h. es kann eine Vorrichtung konstruiert werden, mit der dieses Verfahren automatisch durchgeführt werden kann. Aus strahlenschutztechnischen Gründen ist dies bevorzugt, da die Strahlenbelastung und die Kontaminierung von Menschen bei einem vollautomatischen Verfahren niedrig ist. Da der Proliferationsmarker in hoher Reinheit erhältlich ist, wenn er ausgehend von der erfindungsgemäßen Verbindung synthetisiert wird, kann er dem Patienten entweder sofort oder ohne weitere aufwendige Reinigungsschritte verabreicht werden, ohne daß durch eventuelle Verunreinigungen unerwünschte Wirkungen beim Patienten auftreten. Aufgrund der hohen Ausbeute, in der der Proliferationsmarker erhältlich ist, können bei einer Radiosynthese ausreichende Menge an Marker hergestellt werden, um mehrere Patienten zu untersuchen.

Das folgende Beispiel erläutert die Erfindung näher, ohne sie jedoch darauf einzuschränken.

### Beispiel 1: Synthese der erfindungsgemäßen Verbindung 1-(3'-O-mesyl-2,5'-anhydro-β-D-lyxofuranosyl)thymin

### Experimenteller Teil

### Material und Methoden

### Allgemeiner analytischer Teil

Schmelzpunkte sind unkorrigiert (Büchi, Flawil, Schweiz Modell "535"). Die Elementaranalysen wurden im Organisch Chemischen Institut der Universität Heidelberg angefertigt. Massenspektren (FAB) wurden mit einem Jeol JMS-SX-102A aufgenommen. Kernresonanzspektren (¹H-, ¹³C-NMR) wurden mit einem Bruker AM-500 oder einem Bruker AC-250 Spektrometer aufgenommen (Referenzsubstanz; Tetramethylsilan).

### Chemikalien und Geräte

Chemikalien und Lösungsmittel, auch wasserfreie, wurden von Aldrich® (Deisenhofen, Deutschland) oder Merck® (Darmstadt, Deutschland) bezogen. Die Lösungsmittel waren p.a. Qualität und wurden ohne weitere Aufreinigung eingesetzt. Die Säulenchromatographie wurde an Silica gel 60 (230-400 mesh, Fluka®; Sigma-Aldrich®, Deisenhofen, Deutschland) ausgeführt. Die Dünnschichtchromatographieplatten (Polygram® SIL G/UV254 nm) wurden von Macherey-Nagel (Dueren, Deutschland) bezogen.

### High Performance Liquid Chromatography

Die semipräparative Reinigung des Rohproduktes [¹⁸F]FLT erfolgte mit HPLC unter Verwendung einer RP C18 Säule vom Typ "LUNA" (250 x 21 mm; 5 µ,) von Phenomenex (Torrance; CA, USA). Bedingungen: isokratisch; Ethanol/Wasser = 7,5/92,5 (v/v); Fluß: 10 ^{ml}/ₘᵢₙ. Es wurde ein inline Filter, "Typ, A-410" (2 µm) von Upchurch Scientific (Oak Harbor, USA) benutzt. Radioaktivitätsdetektor: Beckman Coulter "model 170" (Fullerton, CA, USA). Die automatisierte Aufgabe auf die HPLC Säule erfolgte mittels eines 6-Wege Motor Ventils (Besta "model 7060L" Wilhelmsfeld, Germany).
Analytische HPLC Komponenten: Radioaktivitätsdetektor: BIOSCAN Typ, flowcount A' mit Diode (Macarthur, NW, Washington, DC). Säule: ,LUNA, (250 x 4,6 mm, 5 µ; C18); Phenomenex, (Torrance; CA, USA). Bedingungen: isokratisch; Ethanol/Wasser = 7,5/92,5 (v/v); Fluß: 1 ^{ml}/ₘᵢₙ.

### Radioaktivitätsmessungen:

### Capintec Radioisotope Calibrator CRC-2N (Pittsburgh, PA USA)

Das [¹⁸F]Fluorid lieferte das Scanditronix "MC 32 MI" Zyklotron des Deutschen Krebsforschungszentrums (DKFZ, Heidelberg, Germany) über die ¹⁸O(*p*, *n)* ¹⁸F Reaktion unter Verwendung eines [¹⁸O] Wasser-Targets. Das [¹⁸F]Fluorid wurde mit einer CHROMAFIX ® - 30 PS-HCO₃' Kartusche (Macherey-Nagel, Düren, Germany) abgetrennt und mit 0,2 ml 0,05 M K₂CO₃-Lsg als wäßrige Kalium [¹⁸F]fluoridlösung eluiert.

### Synthese von 5'-O-Tritylthymidin (2)

Thymidin (1) (5.15g; 21.26 mmol) wird in 100 ml trockenem Pyridin gelöst und unter Inertgas mit Tritylchlorid (7,02 g; 25,18 mmol) 30 Minuten unter Rückfluß gerührt. Die Reaktionsmischung wird vorsichtig in 1,5 I Eiswasser gegossen und weitere 30 min. gerührt. Der gebildete Niederschlag wird abgesaugt. Der größte Teil der Restfeuchtigkeit wird durch zweimalige azeotrope Trocknung mit Ethanol am Rotationsverdampfer entfernt. Anschließend wird der Feststoff unter Oelpumpenvakuum getrocknet.

Die Umkristallisation aus einer BenzoVAceton Mischung ergibt (2) in einer Ausbeute von 8,60 g (17,75 mmol 83,5 % d. Th.).

Analytische Daten für **(2): mp.** 133,0 - 136,9 °C; **TLC** (*V*_{MeOH}: *V* _{CH2Cl2} = 1:19) R_{f} = 0.29; ^{**1**}**H NMR** (CDCl₃).= **9.36** (s, 1H, N-*H*), **7.56** (q, ⁴ *J* = 1.1 Hz, 1H, G-*H*), **7.44-7.19** (m, 15H, Tr-*H*), **6.42** (dd, ³*J* _{1'-} _{H,2'-Hx}= 8.1 Hz, ³*J*_{1*-H,2'-He} = 5.9 Hz, 1H, 1'-*H*), **4.62-4.51** (m, 1H, 3'-*H*), **4.07** (ddd, ³*J*_{4'-H,5'-H} = 2.9 Hz, ³*J*_{4'-} _{H.5*-H}= 2.9 Hz, ³*J*_{4'-H,3'-H} = 2.9 Hz, 1H, 4'-*H*), **3.45** (dd, ²*J* = 10.3 Hz, ³*J* = 2.9 Hz, 1H, 5*-*H*), **3.36** (dd, ²*J* = 10.5 Hz, ³*J* = 3.1 Hz, 1H, 5'-*H*), **3.07** (d, ³*J* = 4.4 Hz, 1H, 3'-O-*H*), **2.44** (ddd, ²*J* = 13.5 Hz, ³*J*_{2'-He,1'-H} = 5.8 Hz, ³*J*_{2'-He,3'-H} = 2.8 Hz, 1H, 2'-*He*), **2.29** (ddd, ²*J* = 13.7 Hz, ³*J*_{2'-Hx,1'-H} = 7.6 Hz, ³*J*_{2'-Hx,3'-H} = 6.2 Hz, 1H, 2'-Hx), **1.47** (d, ⁴*J* = 1.1 Hz, 3H, 5-*CH*₃); **LRMS** (FAB⁺) ber. für C₂₉H₂₈O₅N₂Na [M+Na]⁺ 507 gef.: 507, ber. für: C₂₉H₂₉O₅N₂ [M+H]⁺ 485 gef.: 485, ber. für: C₂₉H₂₈O₅N₂ [M]⁺ 484 gef.: 484

### Synthese von 1-(5'-O-Trityl-2-deoxy-β-D-lyxofuranosyl)thymin (3)

5'-*O*-Tritylthymidin **(2)** (31,65g; 65,32 mmol) wird in trockenem THF unter Inertgas und rühren gelöst. Nach Kühlen auf -10°C wird Triethylamin (21,0 ml) und dann innerhalb 5 min. portionsweise Mesylchlorid (7,0 ml) zugegeben und weitere 30 Min. unter Kühlung gerührt. Anschließend werden 60 ml Wasser, 60 ml Ethanol und 100 ml 1 M NaOH-Lsg. zugegeben und die Reaktionsmischung unter Rückfluß 90 Min. gerührt Danach werden 100 ml 10 M NaOH-Lsg. zugegeben und weitere 45 min unter Rückfluß gehalten. Das Lösungsmittel wird abgezogen und der Rückstand mit Wasser versetzt. Die wäßrige Phase wird zweimal mit 400 ml Ethylacetat extrahiert und die organische Phase mit Magnesiumsulfat getrocknet. Hierbei entsteht ein weißer Niederschlag, der nach Abfiltration in Dichlormethan/Ethylacetat (Vol. 1/1) aufgeschlämmt und nochmals abfiltriert wird. Die vereinigten organischen Phasen werden eingeengt, auf 30 g Kieselgel aufgezogen und chromatographiert. Eluent: *V*_{MeOH}: *V*_{*CH2Cl2*} = 1:19 mit 0.1 % Triethylamine.
Ausbeute an **(3)**: 20,0 g; (41,275 mmol; 63,2% d. Th.)

Analytische Daten für **(3)**:**mp.** 246,0 - 248,0 °C; **TLC** (*V*_{MeOH}:*V*_{CH2Cl2} = 1:19) R, = 0,33; ^{**1**}**H NMR** (CDCl₃). = **9.09** (s, b, 1H, N-*H*), **7.61** (q, ⁴*J =* 0.8 Hz, 1H,6-*H*), **7.51-7.20** (m, 15H, Tr-*H*), **6.18** (dd, ³*J*_{1'-} _{H,2'-Hx} = 8.3 Hz, ³*J*_{1'-H,2'-He} = 2.3 Hz, 1H, 1'-*H*), **4.48-4.40** (m, 1H, 3'-*H*), **4.03** (ddd, ³*J*_{4'-H,5'-H} = 5.3 Hz, ³*J*_{4'-} _{H,5'-H} = 5,3 Hz, ³*J*_{4'-H,3'-H} = 3.2 Hz, 1H, 4'-*H*), **3.64** (dd, ²*J* = 10.2 Hz,³*J* =5.1 Hz, 1H, 5'-*H*), **3.49** (dd, ²*J* = 10.2 Hz, ³*J* = 5.5 Hz, 1H, 5'-*H*), **3.10** (s, b, 1H, 3'-O*-H*), **2.56** (ddd, ²*J* = 14.9 Hz, ³*J*_{2'-Hx,1'-H} = 8.4 HZ, ³*J*_{2'-} _{Hx,3'-H} = 5.4 Hz, 1H, 2'-*Hx*), **2.17** (dd, ²*J* = 15.3 Hz, ³*J*_{2'-He,1'-H} = 2.1 Hz, 1H, 2'-*He*), **1.76** (d, ⁴*J* = 1.3 Hz, 3H, 5-*CH*₃); **LRMS** (FAB⁺) ber. für C₂₉H₂₈O₅N₂Na [M+Na]⁺ 507 gef. 507. ber. für C₂₉H₂₉O₅N₂ [M+H]⁺ 485 gef. 485, ber. für C₂₉H₂₈O₅N₂ [M]⁺ 484 gef. 484.

### Synthese von 1-(3'-O-Acetyl-2-deoxy-β-D-lyxofuranosyl)thymin (4)

1-(5'-*O*-Trityl-2-deoxy-β-D-lyxofuranosyl)thymin **(3)** (19,5 g; 0,0402 mol) wird in einem 1l Einhalskolben mit Gasansatz in 270 ml trockenem Pyridin unter Inertgas gelöst Unter Eisbadkühlung wird mittels Tropftrichter Acetanhydrid (44 ml; 47,52 g; 0,4655 mol; 11,5 eq.) zugegeben und nach Zugabe 30 min. am Rückfluß gehalten. Anschließend werden 400 ml 80%ige Essigsäure zugesetzt und weitere 2 Stunden unter Rückfluß erhitzt. Das Lösungsmittel wird am Rotationsverdampfer abgezogen, der Rückstand in Dichlormethan aufgenommen, auf Kieselgel aufgezogen und säulenchromatographisch aufgereinigt. Eluent V_{CH2Cl2}/ V_{MeOH} = 95/5
Nach Einrotieren der entsprechenden Fraktionen verbleibt ein schwach gelber, schaumiger Feststoff. Ausbeute an **(4)**: 7,96 g; (28,00 mmol; 70 % d. Th.)

### Synthese von 1-(3'-O-Acetyl-5'-O-Mesyl-2-deoxy-β-D-lyxofuranosyl)thymin (5)

1-(3'-O-Acetyl-2-deoxy-β-D-lyxofuranosyl)thymin **(4)** (7,56 g; 26,5953 mmol) werden unter Inertgas und Eisbadkühlung in 100 ml wasserfreiem Dichlormethan gelöst. Es werden (11,1 ml; 80,077 mmol) Triethylamine und anschließend portionsweise Mesylchlorid (6,2 ml; 80,1047 mmol) zugegeben und eine Stunde gerührt. Das Lösungsmittel wurde abgezogen der Rückstand auf Kieselgel aufgezogen und säulenchromatographisch aufgereinigt. Eluent V_{CH2Cl2}/ V_{MeOH} = 95/5
Nach Einrotieren der entsprechenden Fraktionen verbleibt ein braunes Öl.
Ausbeute an **(5)**: 7,3 g; (18,52 mmol; 65,8 % d. Th.)

### Synthese von 1-(3'-O-Acetyl-5'-lodo-2-deoxy-β-D-lyxofuranosyl)thymin (6)

1-(3'-*O*-Acetyl-5'-*O*-Mesyl-2-deoxy-β-D-lyxofuranosyl)thymin **(5)** (10,2 g; 28,155 mmol) werden unter Inertgas in 180 ml wasserfreiem Aceton in einem 100 ml fassenden, ausgeheizten, druckfesten Glasreaktor gelöst. Natriumiodid (30.12 gr 200,947 mmol) wird zugegeben und der geschlossene Reaktor wird für 8 Stunden auf 110°C erhitzt Die gebildete rotbraune Suspension wird mit Kieselgel versetzt, das Lösungsmittel abgezogen und der Rückstand über Nacht am Ölpumpenvakuum getrocknet. Die anschließende säulenchromatographische Reinigung ergab 7,3 g eines bräunlichen Öles. Eluent: V_{CH2Cl2}/ V_{MeOH} = 95/5
Ausbeute an **(6)**: 7,3 g; (18,52 mmol; 65,8 % d. Th.)

### Synthese von 1-(3'-O-Acetyl-2,5'-anhydro-β-D-lyxofuarnosyl)thymin (7)

1-(3'-*O*-Acetyl-5'-lodo-2-deoxy-β-D-lyxofuranosyl)thymin **(6)** (5,16 g; 13,09 mmol) wird in 300 ml wasserfreiem Acetonitril gelöst. Silberacetat (11,03 g; 66,08 mmol; 5 eq.) wird in einem 500 ml Einhalskolben mit Gasansatz ausgeheizt und unter Inertgas wird die 1-(5'-iodo-3'-*O*-acetyl-2-deoxy-β-D-lyxofuranosyl)thymintösung dazugegeben. Die Suspension wird 5,5 Stunden am Rückfluß gehalten, danach werden weitere 2,18 g Silberacetat zugegeben und nochmals 1 Stunde unter Rückfluß gekocht. Die Reaktionsmischung wird mit 15g Kieselgel versetzt, das Lösungsmittel abgezogen und der Rockstand chromatographiert. Eluent: V_{CH2Cl2}/ V_{MeOH} = 85/15
Nach Entfernung des Lösungsmittels und Trocknung am Hochvakuum verbleiben 2,24 g braunes Öl. Ausbeute an **(7)**: 2,24 g; (8,41 mmol; 64,3 % d. Th.)

### Synthese von 1-(2,5'-anhydro-β-D-lyxofuranosyl)thymine (8)

1-(3'-*O*-Acetyl-2,5'-anhydro-β-D-lyxofuranosyl)thymin **(7)** (2,74 g; 10,29 mmol) wird in 200 ml einer Mischung aus Ethanol/Wasser/Triethlyamin = 4/2/1 gelöst und 5 Stunden bei Raumtemperatur gerührt. Das Lösungsmittel wird am Rotationsverdampfer abgezogen, der Rückstand auf Kieselgel aufgezogen und säulenchromatographisch aufgereinigt. Eluent V_{CH2Cl2}/ V_{MeOH} = 50/50
Nach Entfernung des Lösungsmittels und Trocknung am Hochvakkuum verbleiben 1,7 g beiger, amorpher Feststoff.
Ausbeute an **(8)**: 1,7 g; (7,58 mmol; 73,7% d. Th.)

### Synthese von 1-(3*-O-mesyl-2,5*-anhydro-β-D-lyxofuranosyl)thymin (9)

1-(2,5'-Anhydro-β-D-lyxofuranosyl)thymine **(8)** (1,64 g; 7,314 mmol) wird in einem ausgeheizten 250 ml Einhalskolben mit Gasansatz unter Inertgas in trockenem Dichlormethan gelöst und unter Rühren und Eisbadkühlung zunächst mit Triethylamin (5,1 ml; 3,7 g; 36,571 mmol), anschließend portionsweise mit Mesylchlorid (2,8 ml; 4,189 g; 36,571 mmol) versetzt und auf Raumtemperatur erwärmen lassen. Nach einer Stunde rühren bei RT werden weitere 1 ml TEA und 0,5 ml Mesylchlorid zugesetzt und 45 min. rühren lassen. Nach Abrotieren des Lösungsmittels wird der Rückstand auf Kieselgel aufgezogen und chromatographiert Nach Entfemung des Lösungsmittels und Trocknung am Hochvakuum verbleiben 270,4 mg weißer, kristalliner Feststoff.
Ausbeute an **(9)**: 270,4 mg; (0,894 mmol; 12,2 % d. Th.; MG: = 302,3 g)

## Patentansprüche

1. Verbindung der Formel (1) 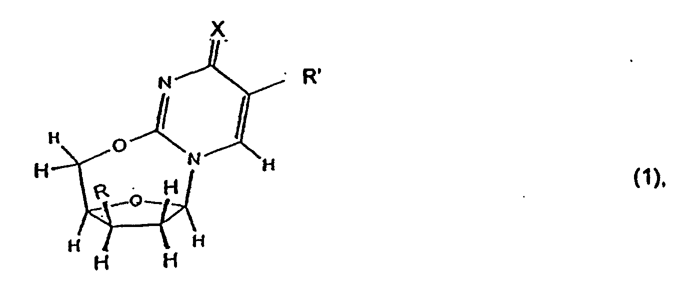 in der
R für Br, J oder R¹-SO₃, wobei R¹ ein unsubstituiertes oder substituiertes C₁-C₅-Alkyl oder ein unsubstituiertes oder substituiertes Phenyl bedeutet;
X für O oder NR", wobei R" eine übliche Schutzgruppe für N bedeutet, und
R¹ für Wasserstoff, Halogen, jeweils substituiertes oder unsubstituiertes C₁-C₇-Alkyl, C₂-C₇-Alkenyl oder C₂-C₇-Alkinyl steht.

2. Verbindung nach Anspruch 1, wobei der Substituent am C₁-C₅-Alkyl eine elektronenziehende Gruppe ist.

3. Verbindung nach Anspruch 1, wobei der Substituent am Phenyl ein C₁-C₅-Alkyl oder eine elektronenziehende Gruppe ist.

4. Verbindung nach einem der vorhergehenden Ansprüche, wobei R¹-SO₃ Methansulfonyl, 4-Nitrophenylsulfonyl, p-Toluensulfonyl oder Trifluormethansulfonyl umfaßt.

5. Verbindung nach einem der vorhergehenden Ansprüche, die 1-(3'-*O*-Mesyl-2,5'-anhydro-β-D-lyxofuranosyl)thymin, 1-(3'-*O*-mesyl-2,5'-anhydro-β-D-lyxofuranosyl)uridin oder 1-(3'-*O*-mesyl-2,5'-anhydro-β-D-lyxofuranosyl)cytidin ist.

6. Verfahren zur Herstellung der Verbindung nach einem der Ansprüche 1 bis 5, wobei in eine Verbindung der Formel (2) 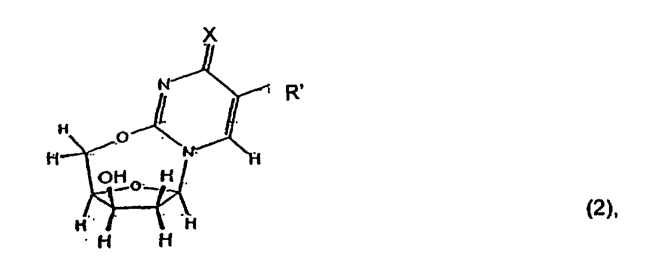 in der X und R' wie vorstehend definiert sind, der Rest R eingeführt wird, wobei R die vorstehenden Bedeutungen hat.

7. Verwendung der Verbindung nach einem der Ansprüche 1 bis 5 zur Einfügung eines Nucleophils an 3'-Position.

8. Verwendung nach Anspruch 9, wobei das Nucleophil ¹⁸F⁻ ist.

## Claims

1. Compound of formula (1) 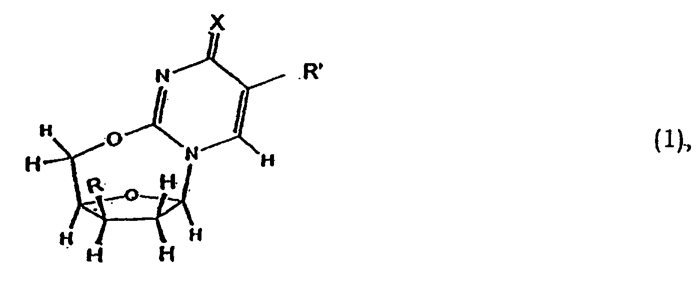 wherein
R represents Br, J or R¹-SO₃, wherein R¹ is an unsubstituted or substituted C₁-C₅-alkyl or an unsubstituted or substituted phenyl;
X is O or NR", wherein R" is a usual protective group for N, and
R¹ represents hydrogen, halogen, respectively substituted or unsubstituted C₁-C₇-alkyl, C₂-C₇-alkenyl, or C₂-C₇-alkinyl.

2. Compound according to claim 1, wherein the substituent on the C₁-C₅-alkyl is an electron-drawing group.

3. Compound according to claim 1, wherein the substituent on the phenyl is C₁-C₅-alkyl or an electron-drawing group.

4. Compound according to one of the preceding claims, wherein R¹-SO₃ comprises methane sulfonyl, 4-nitrophenylsulfonyl, p-toluene sulfonyl or trifluoromethane sulfonyl.

5. Compound according to one of the preceding claims, which is 1-(3'-O-mesyl-2,5'-anhydro-β-D-lyxofuranosyl)-thymine, 1-(3'-O-mesyl-2,5'-anhydro-β-D-lyxofuranosyl)-uridine or 1-(3'-O-mesyl-2,5'-anhydro-β-D-lyxofuranosyl)-cytidine.

6. Method for producing the compound according to one of claims 1 to 5, wherein, in a compound of formula (2) 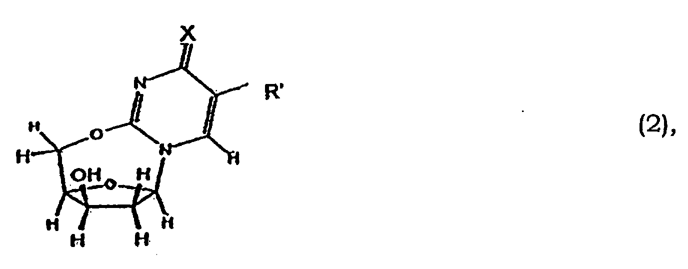 in which X and R' are defined as above, the residue R is introduced, wherein R is as defined above.

7. Use of the compound according to one of claims 1 to 5 for insertion of a nucleophile at the 3' position.

8. Use according to claim 9, wherein the nucleophile is ¹⁸F.

## Revendications

1. Composé de formule (1) 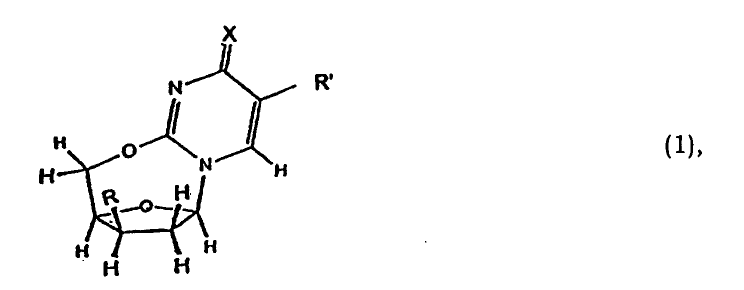 dans laquelle
R est Br, I ou R¹-SO₃, où R¹ représente un groupe alkyle en C₁ à C₅ non substitué ou substitué ou bien un groupe phényle non substitué ou substitué;
X est O ou NR", où R" représente un groupe protecteur usuel pour N, et R' est l'hydrogène, un halogène, ou un groupe alkyle en C₁ à C₇, alcényle en C₂ à C₇ ou alcynyle en C₂ à C₇ chacun substitué ou non substitué.

2. Composé selon la revendication 1, dans lequel le substituant sur le groupe alkyle en C₁ à C₅ est un groupe accepteur d'électrons.

3. Composé selon la revendication 1, dans lequel le substituant sur le groupe phényle est un groupe alkyle en C₁ à C₅ ou un groupe accepteur d'électrons.

4. Composé selon l'une des revendications précédentes, dans lequel R¹-SO₃ comprend les groupes méthanesulfonyle, 4-nitrophénylsulfonyle, p-toluènesulfonyle ou trifluorométhanesulfonyle.

5. Composé selon l'une des revendications précédentes, qui est la 1-(3'-O-mésyl- 2,5'-anhydro-β-D-lyxofuranosyl)thymine, la 1-(3'-O-mésyl-2,5'-anhydro-β-D-lyxofuranosyl)uridine ou la 1-(3'-O-mésyl-2,5'-anhydro-β-D-lyxofuranosyl)cytidine.

6. Procédé de préparation du composé selon l'une des revendications 1 à 5, dans lequel, dans un composé de formule (2) 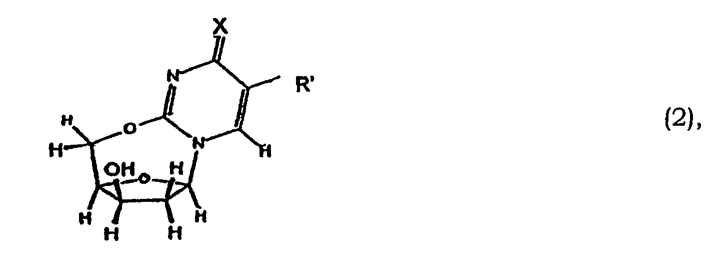 dans laquelle X et R' sont tels que définis précédemment, on introduit le résidu R, où R a les significations ci-dessus.

7. Utilisation du composé selon l'une des revendications 1 à 5 pour insérer un nucléophile en position 3'.

8. Utilisation selon la revendication 9, dans laquelle le nucléophile est ¹⁸F⁻.
